# EUROPEAN PATENT APPLICATION

(11) **EP 2 196 447 A1**
(43) Date of publication of application: **16.06.2010**
(21) Application number: 08253988.3
(22) Date of filing: 12.12.2008
(51) Int. Cl.: C07C 29/149, C07C 29/16, C07C 31/02

(54) **An improved process for hydrogenating alkyl ester(s) in the presence of carbon monoxide**

(71) Applicant: BP p.l.c., London SW1Y 4PD (GB)
(72) Inventor: The designation of the inventor has not yet been filed
(74) Representative: De Kezel, Eric

(57) **Abstract**

The present invention relates to the hydrogenation of an alkyl ester(s) and H₂ stream to produce an alcohol(s) stream. In particular, the present invention relates to a hydrogenation process of an alkyl ester(s) and H₂ stream to produce an alcohol(s) stream, in the presence of:
- a copper-based hydrogenation catalyst that contains manganese; and
- carbon monoxide.

## Description

The present invention relates to the hydrogenation of an alkyl ester(s) and H₂ stream to produce an alcohol(s) stream. In particular, the present invention relates to a hydrogenation process of an alkyl ester(s) and H₂ stream to produce an alcohol(s) stream, in the presence of:
- a copper-based hydrogenation catalyst that contains manganese; and
- carbon monoxide.

Furthermore, the present invention relates to the use of carbon monoxide for increasing the activity of a copper-based hydrogenation catalyst within a process for hydrogenating alkyl ester(s).

In recent years increased use and demand for alcohols such as methanol, ethanol and higher alcohols has led to a greater interest in processes relating to alcohol production. The said alcohols may be produced by the fermentation of, for example, sugars and/or cellulosic materials.

Alternatively alcohols, such as ethanol, may be produced from synthesis gas. Synthesis gas refers to a combination of H₂ and carbon oxides produced in a synthesis gas plant from a carbon source such as natural gas, petroleum liquids, biomass and other carbonaceous materials including coal, recycled plastics, municipal wastes, or any organic material. Thus, alcohol and alcohol derivatives may provide non-petroleum based routes for the production of valuable chemicals and fuels.

Generally, the production of alcohols, for example methanol, takes place via three process steps: synthesis gas preparation, methanol synthesis, and methanol purification. In the synthesis gas preparation step, an additional stage may be employed whereby the feedstock is treated, e.g. the feedstock is purified to remove sulphur and other potential catalyst poisons prior to being converted into synthesis gas. This treatment can also be conducted after synthesis gas preparation; for example, when coal or biomass is employed.

The reaction to produce alcohol(s) from synthesis gas is generally exothermic. The formation of C₂ and C₂₊ alcohols is believed to proceed via the formation of methanol for modified methanol catalysts and cobalt molybdenum sulphide catalysts. However, the production of methanol is equilibrium-limited and thus requires high pressures in order to achieve viable yields. Hence, pressure can be used to increase the yield, as the reaction which produces methanol exhibits a decrease in volume, as disclosed in US 3326956.

A low-pressure, copper-based methanol synthesis catalyst is commercially available from suppliers such as BASF, Johnson Matthey, and Haldor-Topsoe. Methanol yields from copper-based catalysts are generally over 99.5% of the converted CO+CO₂ present. Water is a by-product of the conversion of CO₂ to methanol and the conversion of CO synthesis gas to C₂ and C₂+ oxygenates. In the presence of an active water-gas shift catalyst, such as a methanol catalyst or a cobalt molybdenum catalyst the water equilibrates with the CO to give CO₂ and H₂. A paper entitled, "Selection of Technology for Large Methanol Plants," by Helge Holm-Larsen, presented at the 1994 World Methanol Conference, Nov. 30-Dec. 1, 1994, in Geneva, Switzerland, reviews the developments in methanol production and shows how further reduction in costs of methanol production will result in the construction of very large plants with capacities approaching 10,000 t per day.

Other processes for the production of C₂ and C₂₊ alcohol(s), include the processes described hereinafter;

WO 8303409 describes a process whereby ethanol is produced via the carbonylation of methanol by reacting CO in the presence of a carbonylation catalyst to form ethanoic acid, where the ethanoic acid is then converted to an ethanoate ester, which is then hydrogenated to give ethanol or a mixture of ethanol and another alcohol which can be separated by distillation. Carbonylation can be effected using a CO/H₂ mixture and hydrogenolysis can similarly be conducted in the presence of CO, leading to the possibility of circulating gas between the carbonylation and hydrogenolysis zones with synthesis gas, preferably a 2:1 H₂:CO molar mixture being used as make up gas.

US 4122110 relates to a process for manufacturing alcohols, particularly linear saturated primary alcohols, by reacting CO with H₂ at a pressure between 2 and 25 MPa and a temperature between 150 and 400 °C, in the presence of a catalyst, **characterized in that** the catalyst contains at least 4 essential elements: (a) copper (b) cobalt (c) at least one element M selected from chromium, iron, vanadium and manganese, and (d) at least one alkali metal.

US 4831060 relates to the production of mixed alcohols from CO and H₂ gases using a catalyst, with optionally a co-catalyst, wherein the catalyst metals are molybdenum, tungsten or rhenium, and the co-catalyst metals are cobalt, nickel or iron. The catalyst is promoted with a Fischer-Tropsch promoter like an alkali or alkaline earth series metal or a smaller amount of thorium and is further treated by sulphiding. The composition of the mixed alcohols fraction can be selected by selecting the extent of intimate contact among the catalytic components.

Journal of Catalysis, 1988, 114, 90-99 discloses a mechanism of ethanol formation from synthesis gas over CuO/ZnO/Al₂O₃. The formation of ethanol from CO and H₂ over a CuO/ZnO methanol catalyst is studied in a fixed-bed microreactor by measuring the isotopic distribution of the carbon in the product ethanol when isotopically-enriched ¹³C methanol was added to the feed.

As the importance of alcohols, such as ethanol are ever increasing in today's world, so is the need and desire to produce alcohols with a higher conversion rate and an improved selectivity and productivity. Hence, the present invention relates to the discovery that the addition of a specific amount of carbon monoxide to an alkyl ester and H₂ feed stream actually improves the activity of the manganese-containing copper-based hydrogenation, increasing the hydrogenation rate and therefore the alcohol (e.g. methanol and ethanol) productivity of the overall process.

This is a significant discovery as previously there was a requirement to minimise the carbon monoxide partial pressure in the hydrogenation unit in order to gain maximum alcohol(s) productivity. This means that not only can the hydrogenation rate and alcohol(s) productivity of the overall process be increased by co-feeding carbon monoxide with H₂ but also that the H₂ (and carbon monoxide) feed for the hydrogenation unit can be sourced directly from the synthesis gas generation unit without the need to adjust the synthesis gas ratio (e.g. using an expensive and complicated cryogenic separation) prior to use.

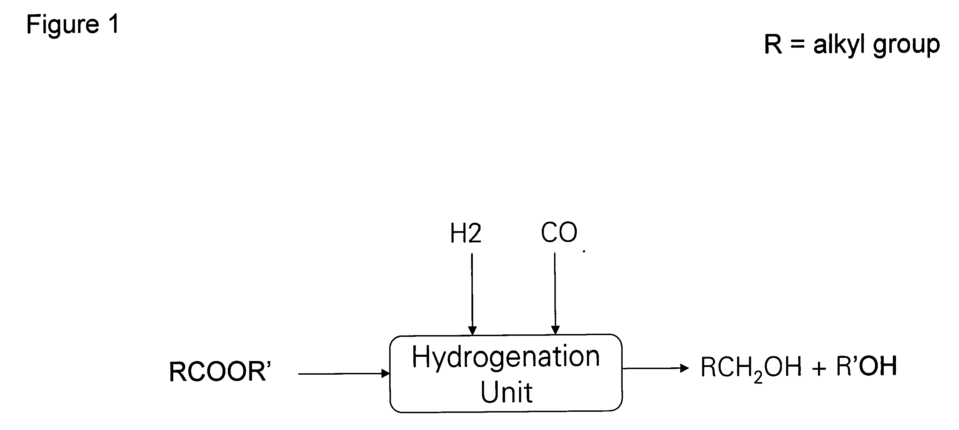

Figure 1 represents an embodiment of a process scheme according to the present invention wherein the references correspond to those used in the present description and appending claims.

Thus, the present invention provides a process for hydrogenating an alkyl ester(s) feed to produce alcohol(s), in the presence of a copper-based hydrogenation catalyst **characterised in that**:
- the feed comprises alkyl ester(s), carbon monoxide and hydrogen; and
- the copper-based hydrogenation catalyst contains manganese.

The present invention also relates to a process for increasing the productivity of a copper-based hydrogenation catalyst in an alkyl ester hydrogenation process, **characterised in that:**
- the process is operated in the presence of carbon monoxide and hydrogen; and
- the copper based hydrogenation catalyst contains manganese.

Furthermore, the present invention relates to the use of carbon monoxide for increasing the activity of a copper-based hydrogenation catalyst, within an alkyl-ester hydrogenation process, wherein said copper-based hydrogenation catalyst contains manganese.

For the purposes of the present invention and appending claims the following terms are defined herewith and hereinafter:
- ' Alkyl ester(s)' refers to a class of chemical compounds and functional groups that consist of a carboxylic acid in which at least one -OH (hydroxy) group is replaced by an -O-alkyl (alkoxy) group; the alkyl ester(s) used according to the present invention are preferably methyl ethanoate, ethyl ethanoate, methyl propanoate, ethyl propanoate, propyl butanoate and butyl pentanoate and preferably emanate from a carbonylation unit (as part of an integrated process to produce alcohols from synthesis gas), wherein they are first purified to remove any carboxylic acids (e.g. ethanoic acid) and water, before being introduced into the hydrogenation unit. However, the alkyl ester(s) may also originate, either solely or in combination, from any other suitable and appropriate source known to those skilled in the art.
- The 'dew point temperature' is a threshold temperature, for example, for a given pure component or mixture of components, at a given pressure, if the system temperature is raised to *above* the dew point temperature, the mixture will exist as a dry gas. Likewise *below* the dew point temperature, the mixture will exist as a vapour containing some liquid.
- 'Gas' and/or 'gas phase' are defined as a pure component, or mixture of components, that are above the dew point temperature.
- 'Gas hourly space velocity' (GHSV) is defined as the volume of gas fed per unit volume of catalyst per hour, at standard temperature (0 °C) and pressure (0.101325 MPa).
- 'Liquid hourly space velocity' (LHSV) is defined as the volume of liquid fed per unit volume of catalyst per hour.

According to the present invention, the reactants i.e. alkyl ester(s) and hydrogen, are introduced into a hydrogenation unit in the presence of a hydrogenation catalyst and carbon monoxide to produce an alcohol(s) containing stream.

Before introduction into the hydrogenation unit the alkyl ester feed contains preferably less than 5 wt% of carboxylic acids (in relation to the total amount of alkyl esters that are being fed to the hydrogenation unit), more preferably less than 1 wt%, even more preferably less than 0.1wt% and most preferably less than 100ppm by wt of carboxylic acids. Before introduction into the hydrogenation unit, the alkyl ester feed contains preferably less than 20 wt% of water (in relation to the total amount of alkyl esters that are being fed to the hydrogenation unit), more preferably less than 2 wt% of water and most preferably less than 0.2 wt% of water.

According to the present invention, the alkyl ester(s) are introduced into the hydrogenation unit together with carbon monoxide and H₂ to produce a stream comprising alcohols.

At least a part, preferably all, of the H₂ fed into the hydrogenation unit emanates directly from a synthesis gas generation procedure (preferably as part of an integrated process for producing alcohols from synthesis gas). In fact, this embodiment represents an additional advantage of the present process, as there is no longer a need to conduct an expensive and complicated separation procedure in order to separate the H₂ from the carbon monoxide following the synthesis gas generation step (i.e. cryogenically separating the H₂ from the carbon monoxide). This is because the applicants have unexpectedly and surprisingly found that the presence of carbon monoxide is actually beneficial towards the activity of the claimed hydrogenation catalyst. Furthermore, the applicants have also unexpectedly found that by having carbon monoxide present it is actually possible to increase the hydrogenation rate and the alcohol(s) (e.g. methanol and ethanol) productivity of the overall process.

According to a preferred embodiment of the present invention, the molar ratio of H₂ to alkyl ester that is introduced into the hydrogenation unit is greater than 1.5:1, preferably the molar ratio is greater than 2:1 and most preferably the molar ratio is greater than 5:1; and is less than 100:1, preferably less than 50:1 and most preferably less than 15:1.

Similarly, the carbon monoxide is also preferably sourced, together with the hydrogen, directly from the synthesis gas feed stream. Again this means that there is no longer a requirement for a complicated and expensive cryogenic separation in order to achieve a H₂ stream with a high purity and a minimal carbon monoxide content.

Preferably the synthesis gas used as the source of the H₂ and carbon monoxide reactant streams is produced from a carbonaceous feedstock.

The carbonaceous feedstock is preferably a material such as biomass, plastic, naphtha, refinery bottoms, crude synthesis gas (from underground coal gasification or biomass gasification), smelter off gas, municipal waste, coal bed methane, coal, and/or natural gas, with coal and natural gas being the preferred sources. To one skilled in the art a combination of sources can also be used, for example coal and natural gas to advantageously increase the H₂ to carbon ratio.

Natural gas commonly contains a range of hydrocarbons (e.g. C₁-C₃ alkanes), in which methane predominates. In addition to this, natural gas will usually contain nitrogen, CO₂ and sulphur compounds. Preferably the nitrogen content of the feedstock is less than 40 mol %, more preferably less than 10 mol % and most preferably less than 2 mol %.

Processes for producing synthesis gas, in a synthesis gas plant, are well known. Each method has its advantages and disadvantages, and the choice of using a particular reforming process over another is governed by economic and available feed stream considerations, as well as by the desire to obtain the optimum (H₂-CO₂):(CO+CO₂) molar ratio in the resulting synthesis gas that is suitable for further chemical processing. A discussion of the available synthesis gas production technologies is provided in both Hydrocarbon Processing, 1999, 78:4, 87-90, and 92-93 and Petrole et Techniques, 1998, 415, 86-93, and are both hereby incorporated by reference.

It is also known that the synthesis gas may be obtained by catalytic partial oxidation of hydrocarbonaceous material in a microstructured reactor as exemplified in IMRET 3: Proceedings of the Third International Conference on Microreaction Technology, ed. W. Ehrfeld, Springer Verlag, 1999, pages 187-196. Alternatively, the synthesis gas may be obtained by short contact time catalytic partial oxidation of hydrocarbonaceous feedstocks as described in EP 0303438. The synthesis gas can also be obtained via a 'compact reformer' process as described in Hydrocarbon Engineering, 2000, 5:5, 67-69; Hydrocarbon Processing, 2000, 79:9, 34; Today's Refinery, 2000, 15:8, 9; WO 9902254; and WO 0023689.

Typically, for commercial synthesis gas production the pressure at which the synthesis gas is produced from a steam reformer ranges from approximately 0.1 to 10 MPa, preferably 2 to 3 MPa and the temperatures at which the synthesis gas exits the reformer ranges from approximately 700 to 1000 °C. Likewise, for commercial synthesis gas production the pressure at which the synthesis gas is produced from an auto-thermal reformer ranges from approximately 0.1 to 10 MPa, preferably 2 to 5 MPa and the temperatures at which the synthesis gas exits the reformer ranges from approximately 700 to 1300 °C. Where the high temperatures are necessary in order to produce a favourable equilibrium for synthesis gas production, and to avoid metallurgy problems associated with carbon dusting. The synthesis gas contains a molar ratio of (H₂-CO₂):(CO+CO₂) ranging from 0.8 to 3.0, which is dependent on the carbonaceous feedstock(s) and the method of reforming used. For example, when natural gas is used as the carbonaceous feedstock for steam reforming, the synthesis gas obtained usually has a maximum (H₂-CO₂):(CO+CO₂) ratio of 3.0. However, when natural gas is used as the carbonaceous feedstock for autothermal reforming, the synthesis gas obtained usually has a (H₂-CO₂):(CO+CO₂) ratio of 1.5. Synthesis gas generated from coal gasification is a carbon monoxide rich stream: the water gas shift reaction can be used to tune the H₂:CO molar ratio to meet the needs of the downstream process(es).

According to a preferred embodiment of the present invention, the molar ratio, (H₂-CO₂):(CO+CO₂), of the synthesis gas stream exiting the synthesis gas generation unit(s) is greater than 1.6, more preferably greater than 1.8 and most preferably greater than 2.0. Preferably, the molar ratio, (H₂-CO₂):(CO+CO₂), of said synthesis gas stream exiting the synthesis gas generation unit(s) is less than 3.0, preferably less than 2.75, more preferably less than 2.4 and most preferably less than 2.2.

According to the present invention, carbon monoxide represents more than 1 mol%, preferably more than 10 mol% and most preferably more than 20 mol% of the total synthesis gas fed to the hydrogenation unit; and less than 90 mol%, preferably less than a 80 mol% and most preferably less than 50 mol% of the total synthesis gas fed to the hydrogenation unit.

As mentioned hereinabove, the H₂ feed is preferably sourced directly from the synthesis gas feed without further treatment, however, if need be, the H₂ content of the synthesis gas can be further increased by subjecting the said synthesis gas to a water gas shift reaction.

Alternatively the H₂ stream may originate from a variety of other chemical processes, including ethene crackers, styrene manufacture and catalytic reforming. However, it is known that the main commercial processes for purposeful generation of H₂ are autothermal reforming, steam reforming and partial oxidation of hydrocarbonaceous feedstocks such as natural gas, coal, coke, deasphalter bottoms, refinery residues and biomass. H₂ may also be produced by electrolysis of water.

The overall choice of technology for producing H₂ is generally determined by the following economic considerations and factors:
i. feedstock cost
ii. feedstock availability
iii. capital cost
iv. local energy and operating costs; and
v. safety and environmental considerations

Similarly, as explained hereinabove, whilst it is preferable to obtain the carbon monoxide and H₂ together directly from the synthesis gas generation unit, the carbon monoxide fed into the hydrogenation unit may also be generated from any other suitable source known to those skilled in the art.

According to the present invention, the reactants (i.e. the alkyl ester(s) and H₂ together with the carbon monoxide) are then introduced into a hydrogenation unit. Preferably, the alkyl ester feed is vaporised before contacting the hydrogenation catalyst, by either:
- heating the alkyl ester in a separate vaporiser before contacting the H₂ and carbon monoxide feed; and/or
- heating the alkyl ester together with the H₂ and carbon monoxide (e.g. either in a separate vaporiser or on a pre-bed to the hydrogenation unit).

Overall, the feed mixture, including recycles, entering into the hydrogenation unit (i.e. the alkyl ester(s) and H₂ together with the carbon monoxide) is preferably more than 10 °C, preferably more than 20 °C above its dew point temperature.

Preferably the hydrogenation unit is operated at high conversion of alkyl ester(s) to alcohol(s), e.g. higher than 50%, more preferably higher than 75%, more preferably still higher than 90% and most preferably higher than 95%.

In addition to the production of alcohol(s), the hydrogenation process also produces other reaction products such as other alkyl ester(s) (arising from trans-esterification), trace amounts of methane, ethane, water, ethers and aldehydes, together with unreacted starting materials, e.g. unreacted alkyl ester(s), and unreacted H₂ and carbon monoxide.

For example, when methyl ethanoate is used as a feed for the hydrogenation process, in addition to producing methanol and ethanol, it also produces ethyl ethanoate by trans-esterification. The proportion of ethyl ethanoate present in the exit stream will be determined by the nature of the catalyst and the degree of conversion. The proportion of ethyl ethanoate may be further increased, if desired, by introducing an acidic function into the catalyst to promote in situ trans-esterification. This trans-esterification is advantageous, as it reduces the amount of unreacted methyl ethanoate present in the reactor effluent since the methyl ethanoate forms a close boiling point mixture with the methanol, and is thus carried with the methanol recycles either to other process stages (if part of an integrated process for the conversion of synthesis gas to alcohols) and/or as a recycle to the hydrogenation unit. More preferably the quantities of these fluxes are minimised.

The catalyst(s) employed in the hydrogenation unit is a manganese-containing copper-based catalyst (for example a copper chromite, or a mixed copper metal oxide based catalyst, wherein the second metal is manganese and a third metal could be zinc or zirconium).

Preferably the copper-based catalyst(s) employed in the hydrogenation unit contains more than 0.1 wt % and most preferably more than 1 wt % of manganese; and likewise it contains preferably less than 50 wt %, more preferably less than 20wt% and most preferably less than 10 wt% of manganese.

All of the aforementioned catalysts may advantageously be supported on any suitable support known to those skilled in the art; non-limiting examples of such supports include: carbon; silica; titania; clays; aluminas; zinc oxide; zirconia; and mixed oxides; or mixtures thereof. Preferably, the copper based catalyst is supported on alumina.

According to a preferred embodiment of the present invention, the catalyst(s) employed is heterogeneous.

The hydrogenation process may be operated in a gas phase, or a mixed gas/liquid phase regime. The mixed gas/liquid phase is where a proportion of the substrate (e.g. methyl and/or ethyl ethanoate) to be hydrogenated is in the liquid phase. A gas phase regime is the preferred mode of operation. The hydrogenation can be conducted in batch or semi continuous or continuous mode. Continuous mode of operation is the most preferred.

The hydrogenation reaction can be conducted in adiabatic or isothermal mode; where adiabatic mode of operation is preferred. Suitable reactors include single, or a plurality, of adiabatic bed reactors which can be used in series or parallel. For reactors utilised in series, heat exchangers and/or intercoolers and/or additional reactant and/or recycle of intermediates can be employed in between successive reactors to control the reaction temperature. The preferred adiabatic temperature rise is less than 50 °C, preferably less than 25 °C and most preferably less than 10 °C. The preferred use of adiabatic reactors is in series. The adiabatic reactors may be operated at different temperatures depending on composition of the individual reactor feeds.

The hydrogenation can also be conducted in multi-tubular reactors in which case a cooling/heating medium is circulated around the tubes to control the temperature. For exothermic reactions, as such, there will be a radial temperature gradient in the reactor, the preferred gradient is less than 50 °C, preferably less than 25 °C and most preferably less than 10 °C. The preferred flow regime in this type of reactor is turbulent rather than laminar, this corresponds to a Reynolds number greater than 2100 (where the velocity is approximated by velocity in an unpacked tube).

The hydrogenation reaction can also be conducted in other reactor types such as fluidised bed, spinning basket and buss loop, heat exchanger reactors. A mixed liquid/gas phase hydrogenation reaction can be conducted with co-flow or counterflow of the H₂ and gas to the liquid (e.g. a bubble reactor). The preferred mode of operation of gas/liquid reactors is co-flow, also known as trickle bed operation; this can be conducted in at least one tubular and/or multi-tubular reactor in series. The hydrogenation reaction may change from a mixed gas/liquid phase to a fully gas phase reaction, as the reaction proceeds down the reactor. The mixed phase hydrogenation can also be conducted in other types of reactors, or within a combination of different reactors, for example in a slurry or stirred tank reactor with, or without, external circulation and optionally operated as a cascade or stirred tanks, a loop reactor or a Sulzer mixer-reactor.

The hydrogenation reactor(s) preferably operate at a temperature of greater than 150 °C, but less than 290 °C.

According to a preferred embodiment of the present invention the hydrogenation reaction temperature is greater than 150°C, preferably greater than 170°C and most preferably greater than 190°C; and less than 250°C, preferably less than 230°C and most preferably less than 220°C.

The hydrogenation reaction may be operated at a pressure of greater than 1 MPa, more preferably at a pressure of greater than 3 MPa and most preferably at a pressure of greater than 5 MPa; and at a pressure of less than 15 MPa, more preferably at a pressure less than 13 MPa and most preferably at a pressure less than 9 MPa.

According to an embodiment of the present invention, when the hydrogenation unit(s) is operated with a copper-based catalyst, the feed mixture introduced into the reactor(s) is always above its dew point temperature.

The GHSV for continuous operation may be in the range 50 to 50,000 h⁻¹, preferably from 1,000 to 30,000 h⁻¹ and most preferably from 2,000 to 9,000 h⁻¹.

The ester liquid substrate introduced into the hydrogenation unit preferably has an LHSV less than 10 h⁻¹, more preferably less than 5 h⁻¹ and most preferably less than 3 h⁻¹; for example, a typical LHSV for normal operation is approximately 1 h⁻¹.

For the purposes of calculating the GHSV and/or the LHSV of the present invention, when the reactors are arranged in series, said "catalyst volume" refers to the combined volume of the catalyst in all of the reactors in series.

According to an embodiment of the present invention, the applicants have unexpectedly found that a conventional methanol synthesis reactor may alternatively be used as the aforementioned hydrogenation unit in order to co-produce methanol and the said ester hydrogenation product(s).

According to the present invention, the stream exiting the hydrogenation unit is then subjected to a separation stage (e.g. distillation), whereby a fraction comprising alcohol(s) is separated and recovered. For example, said separated and recovered alcohols may comprise any of the following: methanol, ethanol, propanol(s) (n-propanol with low amounts of iso-propanol), butanol(s) (n-butanol and iso-butanol) and pentanol(s).

According to a preferred embodiment of the present invention, the stream exiting the hydrogenation unit may be first subjected to a separation stage, e.g. cooling the effluent and then feeding the cooled effluent to a flash vessel (wherein the said effluent can be both cooled and/or flashed several times). After this treatment, a vapour stream containing the majority of the hydrogen, carbon monoxide and inert gases (including light alkanes; nitrogen; and CO₂) in the effluent is recovered for recycle to the hydrogenation unit(s). The concentration of said inert gases can be controlled by diverting a proportion of the gas recycle stream to purge.

It should be noted that whilst all of the aforementioned temperature and pressure operating conditions form preferred embodiments of the present invention, they are not, by any means, intended to be limiting, and the present invention hereby includes any other pressure and temperature operating conditions that achieve the same effect.

### Examples

Examples 1 to 4 demonstrate the promotional effect of carbon monoxide on the hydrogenolysis of methyl ethanoate using a manganese-containing copper-based catalyst.

### Catalysts

The Catalysts used in these Examples were T-4489 (supplied by Süd-Chemie), which has the following composition: CuO (56wt.%), MnO₂ (10wt.%), Al₂O₃ (34wt.%); and T-2130 (also supplied by Süd-Chemie), which has the following composition: CuO (33wt.%), ZnO (66wt.%).

### Catalyst Testing

The catalyst testing experiments were carried out in a pressure flow reactor. The catalyst was heated to 100 °C under a flow of 5 mol % H₂ in N₂ at 2.5 MPa and a GHSV of 6000 h⁻¹. The concentration of H₂ was increased in stages to 10, 20, 40, 70 and 100 mol % with a 1 h dwell time at each stage. The catalyst was heated at 1 °C/min to a holding temperature of 180 °C and was held for a dwell time of 1 h. At this point catalyst activation was considered complete.

### Example 1

A mixture of carbon monoxide (63.4 vol%), H₂ (31.6 vol%), and methyl ethanoate (5.0 vol%) was passed over T-4489 at 190 °C, with a pressure of 5 MPa and a GHSV of 5400 h⁻¹ for 18 h. The observed conversion of methyl ethanoate was 92.1 % and the selectivity to ethanol and the ethyl portion of ethyl ethanoate was 99.8%. The ethanol productivity is defined as kilograms of ethanol plus kilograms of the ethyl portion of ethyl ethanoate produced per kilogram of catalyst per hour (kg/kg_{cat}/h). The ethanol productivity observed for this experiment was 0.36 kg/kg_{cat}/h.

### Example 2 - Comparative Example

A mixture of nitrogen (63.4 vol%), H₂ (31.6 vol%), and methyl ethanoate (5.0 vol%) was passed over T-4489 at 190 °C, with a pressure of 5 MPa and a GHSV of 5400 h⁻¹ for 18 h. The observed conversion of methyl ethanoate was 67.1%; the selectivity to ethanol and the ethyl portion of ethyl ethanoate was 99.8% and the ethanol productivity, as defined in Example 1, was 0.26 kg/kg_{cat}/h.

### Example 3 - Comparative Example

A mixture of carbon monoxide (63.4 vol%), H₂ (31.6 vol%), and methyl ethanoate (5.0 vol%) was passed over T-2130 at 190 °C, with a pressure of 5 MPa and a GHSV of 5400 h⁻¹ for 18 h. The observed conversion of methyl ethanoate was 9.4%; the selectivity to ethanol and the ethyl portion of ethyl ethanoate was 99.9% and the ethanol productivity, as defined in Example 1, was 0.05 kg/kg_{cat}/h.

### Example 4 - Comparative Example

A mixture of nitrogen (63.4 vol%), H₂ (31.6 vol%), and methyl ethanoate (5.0 vol%) was passed over T-2130 at 190 °C, with a pressure of 5 MPa and a GHSV of 5400 h⁻¹ for 18 h. The observed conversion of methyl ethanoate was 28.1%; the selectivity to ethanol and the ethyl portion of ethyl ethanoate was 99.8% and the ethanol productivity, as defined in Example 1, was 0.14 kg/kg_{cat}/h.

### Results Summary

The results for the Examples are summarised in the Table 1 (shown below). The results for Examples 1 and 2 show that the hydrogenation rate for the manganese-containing copper-based catalyst T-4489 was increased by the presence of carbon monoxide. The results from Examples 3 and 4 show that the same promotional effect was not observed for the representative copper-zinc oxide catalyst T-2130: moreover, the hydrogenation rate for T-2130 was actually decreased by the presence of carbon monoxide.

**Table 1: Results for Examples 1 to 4: the H₂ and methyl ethanoate concentrations are 31.6 vol% and 5.0 vol% respectively in all four Examples.**

| **Catalyst** | **Example** | **CO (vol%)** | **N₂ (vol%)** | **Conversion (%)** | **Selectivity (%)** | **Productivity kg/kg_{cat}/h** |
|---|---|---|---|---|---|---|
| T-4489 | 1 | 63.4 | 0 | 92.1 | 99.8 | 0.36 |
| | 2 | 0 | 63.4 | 67.1 | 99.8 | 0.26 |
| T-2130 | 3 | 63.4 | 0 | 9.4 | 99.9 | 0.05 |
| | 4 | 0 | 63.4 | 28.1 | 99.8 | 0.14 |

## Claims

1. A process for hydrogenating an alkyl ester(s) feed to produce alcohol(s) in the presence of a copper-based hydrogenation catalyst; **characterised in that:**
- the feed comprises alkyl ester(s), carbon monoxide and hydrogen; and
- the copper-based hydrogenation catalyst contains manganese.

2. A process for increasing the productivity of a copper-based hydrogenation catalyst in an alkyl ester hydrogenation process, **characterised in that**:
- the process is operated in the presence of carbon monoxide and hydrogen; and
- the copper based hydrogenation catalyst contains manganese.

3. A process according to any one of the preceding claims, wherein the hydrogen feed that is introduced into the hydrogenation unit together with the said carbon monoxide is sourced directly from a synthesis gas generation unit.

4. A process according to any of the preceding claims, wherein the alkyl ester(s) are methyl ethanoate, ethyl ethanoate, methyl propanoate, ethyl propanoate, propyl butanoate and butyl pentanoate.

5. A process according to any of the preceding claims, wherein the alkyl ester feed contains preferably less than 5 wt% of carboxylic acids (in relation to the total amount of alkyl esters that are being fed to the hydrogenation unit), more preferably less than 1 wt%, even more preferably less than 0.1wt% and most preferably less than 100ppm by weight of carboxylic acids before being introduced into the hydrogenation unit.

6. A process according to any of the preceding claims, wherein the alkyl ester feed contains less than 20 wt% of water (in relation to the total amount of alkyl esters that are being fed to the hydrogenation unit), preferably less than 2 wt% of water and most preferably less than 0.2 wt% of water before being introduced into the hydrogenation unit.

7. A process according to any of the preceding claims, wherein the molar ratio of hydrogen to alkyl ester that is introduced into the hydrogenation unit is greater than 1.5:1, preferably the molar ratio is greater than 2:1 and most preferably the molar ratio is greater than 5:1.

8. A process according to any of the preceding claims, wherein the molar ratio of hydrogen to alkyl ester that is introduced into the hydrogenation unit is less than 100:1, preferably less than 50:1 and most preferably less than 15:1.

9. A process according to any of the preceding claims, wherein carbon monoxide represents more than 1 mol%, preferably more than 10 mol% and most preferably more than 20 mol% of the total synthesis gas fed to the hydrogenation unit.

10. A process according to any of the preceding claims, wherein carbon monoxide represents less than 90 mol%, preferably less than a 80 mol% and most preferably less than 50 mol% of the total synthesis gas fed to the hydrogenation unit.

11. A process according to any of the preceding claims, wherein the alkyl ester feed is vaporised before contacting the manganese-containing copper-based hydrogenation catalyst(s) employed in the hydrogenation unit.

12. A process according to any of the preceding claims, wherein the feed mixture, including recycles, entering into the hydrogenation unit (i.e. the alkyl ester(s) and hydrogen together with the carbon monoxide) is more than 10 °C, preferably more than 20 °C above its dew point temperature.

13. A process according to any of the preceding claims, wherein the hydrogenation unit is operated at high conversion of alkyl ester(s) to alcohol(s); preferably higher than 50%, more preferably higher than 75%, more preferably still higher than 90% and most preferably higher than 95%.

14. A process according to any of the preceding claims, wherein the manganese-containing copper-based hydrogenation catalyst(s) employed in the hydrogenation unit is a copper chromite, or a mixed copper metal oxide based catalyst, wherein the second metal is manganese and a third metal is either zinc or zirconium.

15. A process according to any of the preceding claims, wherein the manganese-containing copper-based hydrogenation catalyst(s) employed in the hydrogenation unit contains more than 0.1 wt % and most preferably more than 1 wt % of manganese.

16. A process according to any of the preceding claims, wherein the manganese-containing copper-based hydrogenation catalyst(s) employed in the hydrogenation unit contains less than 50 wt %, more preferably less than 20wt% and most preferably less than 10 wt% of manganese.

17. A process according to any of the preceding claims, wherein the manganese-containing copper-based hydrogenation catalyst(s) employed in the hydrogenation unit is supported on one of the following: carbon; silica; titania; clays; aluminas; zinc oxide; zirconia; and mixed oxides; or mixtures thereof.

18. A process according to any of the preceding claims, wherein the manganese-containing copper-based hydrogenation catalyst(s) employed in the hydrogenation unit is supported on alumina.

19. A process according to any of the preceding claims, wherein the manganese-containing copper-based hydrogenation catalyst(s) employed in the hydrogenation unit is heterogeneous.

20. The use of carbon monoxide for increasing the activity of a copper-based hydrogenation catalyst, within an alkyl-ester hydrogenation process, wherein said copper-based hydrogenation catalyst contains manganese.
